# EUROPEAN PATENT APPLICATION

(11) **EP 3 034 086 A1**
(43) Date of publication of application: **22.06.2016**
(21) Application number: 14075080.3
(22) Date of filing: 19.12.2014
(51) Int. Cl.: A61K 33/04, A61K 33/06, A61K 36/21, A61K 36/235, A61K 36/28, A61K 36/484, A61P 17/08, A61P 17/10, A61K 8/00

(54) **Topical treatment of acne with extract of herbs combined with minerals**

(71) Applicant: Shmuel Gonen Technologies Ltd., 55286 Kiryat Ono (IL)
(72) Inventor: Gonen, Shmuel, 55286 Kiryat Ono (IL)
(74) Representative: Riccardi, Sergio

(57) **Abstract**

A composition for topical treatment of various forms of acne comprising 5-20% by volume of an ethanol extract of Calendula officinalis plant, 15-20% by volume of an ethanol extract of Glycyrrhiza glabra plant, 15-30% by volume of an ethanol extract of a Foeniculum officinale plant, 15-45% by volume of a saturated water solution of at least one mineral salt selected from at least one of the group consisting of NaCl, KCl and MgCl.sub.2, and 1-3% by volume of an ethanol extract of an Atriplex halimus plant.

## Description

### FIELD OF THE INVENTION

The present invention generally concerns treatment of human skin disorders and more specifically acne vulgaris. Potentially, the invention is also applicable to other, related skin deceases such as seborrhea and scalp psoriasis.

### BACKGROUND OF THE INVENTION

Although the precise causes for the development of acne have not been fully resolved, it has been established that a combination of at-least the following three factors is involved:-- (a) Excessive production of sebum; (b) Blocking of the entrance of the hair follicle canals; and (c) Inflammatory processes caused by excessive development of bacteria.

One of the causes for excessive sebum production is the increased activity of androgenic hormones, which is typical to teenagers.

The inflammatory response is a reaction to the follicle canal blocking by keratin plugs.

Conventional topical treating methods generally fall into five major categories: (a) Application of antiseptics preparations such as various types of soaps and the like detergents. This method is disagreeable due to sever skin drying. (b) Chemical antiseptics such as containing sulfur, which again suffers the disadvantage of skin over-drying. (c) Compositions such as containing salicylic acid. The disadvantage of these methods resides in causing the skin to become light-sensitive. (d) Use of vitamin A derivatives commercially known as ROACCUTANE™, CURATANE™, ISOTREX™ and others, which contain tretinoin as active agent. (e) Antibiotics such as Erythromycin or Benzoyl-Peroxide. Drawbacks and limitations known in connection with other antibiotic medications in the long run may arise including immunity of the body against the efficacy of these drugs.

Oral administered medications have also been tested, mainly in effort to cure the inflammatory of the sebum glands. However, there were found ineffective for removing the white and black heads of the acne comedones.

Attempted use of the new age antibiotics Tetracycline (e.g. MINOCIN™) again, are bound to cause the generally undesirable side-effects of antibiotic treatments.

Another example of orally administered preparations are derivatives of vitamin A, such as ROACCUTANE™. Although reported quite effective, side-effects such as temporary hair loss, sensitivity to sun blaze and joint aches have been experienced; moreover, routine examinations of lever functions must be followed.

Yet another treatment, confined to females only, is based on restricting the sensitivity to the masculine hormone Testosterone.

In extreme cases of inflammation, the use of cortisone has been recommended, albeit for a limited period only.

Experiments with vitamin B or minerals have proved partially successful in reducing sebum production.

For external, night-time application, medications such as DALACIN™, which is based on clindamycin as an active ingredient, have been proposed.

For further relevant background information refer to, U.S. Pat. No. 5,409,917 (Robinson et al).

The closest prior art is represented by the inventor's European Patent EP2101798 B1 relating to a composition for a topical treatment of various forms of acne comprising an effective amount of an ethanol extract of Glycyrrhiza glabra plant, an ethanol extract of a Foeniculum officinale plant, a mineral salt and an ethanol extract of an Atriplex halimus plant for the topical treatment of various forms of acne, the effective amount comprising: (a) 20-40% by volume of an ethanol extract of Glycyrrhiza glabra plant; (b) 15-30% by volume of an ethanol extract of a Foeniculum officinale plant; (c) 15-45% by volume of a saturated water solution of at least one mineral salt selected from at least one of the group consisting of NaCl, KCI and MgCl.sub.2; and (d) 1-3% by volume of an ethanol extract of an Atriplex halimus plant (hereinafter referred to as "the Prior Patent Composition").

### OBJECTS OF THE INVENTION

While treatment results with the prior patent composition provided significant improvement over most conventional methods and compositions, it has been recognized by the inventor that in order to enhance its effectiveness, a stronger anti-inflammatory component should be included.

### PREFERRED EMBODIMENTS OF THE INVENTION

In one embodiment, the composition of the invention contains as active agents the following: (a) The plant CALENDULA OFFICINALIS; (b) The plant GLYCYRRHIZA GLABRA; (c) The plant FOENICULUM OFFICINALE; (d) Mineral salts preferably selected from the group of NaCl, KCI and MgCl.sub.2 (any one or any combination thereof); (e) The plant ATRIPLEX HALIMUS; (f) Alcohol (e.g., ethanol)

In more detail:
A. The CALENDULA OFFICINALIS (also known as "poet's marigold" or "pot marigold") has among its main components triterpene saponins 2-10%, volatile oil and vitamins C and Ca.

The selection of this plant (specifically, its flower head) is based on the recognition that in topical use it is immunostimulating, astringent, and anti-inflammatory.
B. The GLYCYRRHIZA GLABRA is a plant of the PAPILIONACEAE family.

The active ingredients of this plant are GLYCYRRHIZIN and GLYCYRRHIZIN ACID.

The selection of this plant is based on the recognition that it's chemical structure presents phyto-estrogen activity which considerably reduces the effect of testosterone which, in turn, reduces the production of sebum; however, being phyto-estrogen, it does not provoke any side-effects associated with the corticosteroid estrogen, even among males. C. The FOENICULUM OFFICINALE is a plant of the UMBELLIFERAE family.

The selection of this plant is based on the recognition that its chemical structure presents cortico-steroid-like properties such as anti-inflammatory properties and improvement of the relationship between estrogen and testosterone.
D. The selection of mineral salts such as NaCl, KCI, and/or MgGl.sub.2 is based on the recognition that they contribute to the prevention of the development of bacteria by creating higher osmotic pressure gradient between the outside and the inside of the cells and causes drying-out of the affected area and the bacteria.
E. The plant ATRIPLEX HALIMUS (found in arid areas) is salt-rich thus also increasing the osmotic pressure of cells.

In one embodiment, extracts of the CALENDULA OFFICINALIS, the GLYCYRRHIZA GLABRA, the FOENICULUM OFFICINALE, and the ATRIPLEX HALIMUS are prepared by admixing components of the individual plants with a solvent such as 95% ethyl alcohol.

Dry, shredded flower heads of the CALENDULA OFFICINALIS plant are admixed with elemental (powder) at between 2-45% by volume.

Dry, shredded roots of the GLYCYRRHIZA GLABRA plant are admixed with elemental sulfur.

Dry, shredded plant grains of the FOENICULUM OFFICINALE plant are admixed with elemental sulfur.

The ground ATRIPLEX HALIMUS plant is admixed with elemental sulfur.

The admixed extracts are then combined with mineral salts into the final composition. In one embodiment, the overall mineral salts amount to between 1-15% of the composition; the overall volume of alcohol amounts to between 5-60%; and the overall volume of sulfur amounts to between 1-15%.

### PREPARATION EXAMPLES

I--A quantity of 5 lit. of Calendula officinalis extract is prepared as follows:
   (1) Mixing 600 gr. of the CALENDULA OFFICINALIS shred with 250 gr. of sulfur powder in a 5 lit container; (2) Topping the container with ethyl alcohol 95% solvent; and, (3) Stirring the mixture for 15 days.
II--A quantity of 5 lit. of Glycyrrhiza Glabra extract is prepared as follows:
   (1) Mixing 600 gr. of the GLYCYRRHIZA GLABRA shred with 250 gr. of sulfur powder in a 5 lit. container; (2) Topping the container with ethyl alcohol 95% solvent; and, (3) Stirring the mixture for 15 days.
III--A quantity of 5 lit. of Foeniculum Officinale extract is prepared as follows:
   (1) Grinding the plant shred to flour-like powder; (2) Mixing 800 gr. of the plant powder with 250 gr. of sulfur powder in a 5 lit. container; (3) Topping the container with ethyl alcohol 95% solvent; and, (4) Stirring the mixture for 15 days.
IV--A quantity of 5 lit. of Atriplex Halimus extract is prepared as follows:
   (1) Grinding into fine flour-like powder; (2) Mixing 50 gr. of the plant powder with 100 gr. of sulfur powder in a 5 lit. container; (3) Adding 2 lit. of water; (4) Adding 2.5 lit. of ethyl alcohol 95% solvent; and, (5) Stirring the mixture for 15 days.
V--A quantity of 5 lit. of mineral(s) solution is prepared as follows:
   (1) Filling 2.0 Kg. of the minerals NaCl, KCI and MgCl.sub.2 (or any one or any combination thereof) in a 5 lit. container; (2) Adding 1.5 lit. of water; (3) Placing for 5 days for initial dissolution; (4) Adding 100 gr. of sulfur powder under continuous stirring; (5) Topping the container with ethyl alcohol 95% solvent; and (6) Stirring the mixture for 10 days.
VI--A quantity of 5 lit. of the final product are prepared as follows:
   (1) Filling 500 cc of the CALENDULA OFFICINALIS extract into a 5 lit. container; (2) Pouring 1,000 cc of the GLYCYRRHIZA GLABRA extract into the container; (3) Adding 990 cc of the FOENICULUM OFFICINALE extract; (4) Adding 100 cc of the ATRIPLEX HALIMUS extract; (5) Adding 2,000 cc of the mineral(s) solution; (6) Adding 400 cc of ethyl alcohol 95% solvent; (7) Stirring for 20 min. (8) Straining the mixture for removing remaining plants residue or other sediments; and, optionally, (9) Adding about 10 cc of vitamin E and/or yeast.

It will be appreciated by those skilled in the art, that the invention has been described hereinabove with reference to certain examples and specific embodiments. However, these are not the only examples and embodiments in which the invention may be practiced. Indeed, various modifications may be made to the above-described examples and embodiments without departing from the spirit and scope of the present invention, and it is intended that all such modifications be included within the scope of the following claims.

## Claims

1. A composition for topical treatment of various forms of acne comprising: (a) 5-20% by volume of an ethanol extract of Calendula officinalis plant; (b) 15-20% by volume of an ethanol extract of Glycyrrhiza glabra plant; (c) 15-30% by volume of an ethanol extract of a Foeniculum officinale plant; (d) 15-45% by volume of a saturated water solution of at least one mineral salt selected from at least one of the group consisting of NaCl, KCl and MgCl.sub.2; and (e) 1-3% by volume of an ethanol extract of an Atriplex halimus plant.

2. The composition of Claim 1 further comprising 6-10% by volume of a solvent.

3. The composition of Claim 2, wherein the solvent is ethyl alcohol.

4. The composition of Claim 2 further comprising 8-12% by volume of water.

5. The composition of Claim 1, wherein the extract of the Calendula officinalis plant is admixed with 2-45% by volume of sulfur.

6. The composition of Claim 1, wherein the extract of the Glycyrrhiza glabra plant is admixed with 2-45% by volume of sulfur.

7. The composition of Claim 1, wherein the extract of the Foeniculum officinale plant is admixed with 2-45% by volume of sulfur.

8. The composition of Claim 1, wherein the extract of the Atriplex halimus plant is admixed with 2-45% by volume of sulfur.

9. The composition of claim 1, wherein the overall volume of mineral salts is between 5-50%.
